# EUROPÄISCHE PATENTANMELDUNG

(11) **EP 4 628 036 A1**
(43) Veröffentlichungstag der Anmeldung: **08.10.2025**
(21) Anmeldenummer: 25167712.6
(22) Anmeldetag: 01.04.2025
(51) Int. Cl.: A61B 17/70

(54) **HÜLSE FÜR EINE IMPLANTATSCHRAUBE UND IMPLANTATSCHRAUBE MIT EINER HÜLSE**

(30) Priorität: 05.04.2024 DE 102024109618
(71) Anmelder: Aesculap AG, 78532 Tuttlingen (DE)
(72) Erfinder: KRUEGER, Sven, 78647 Trossingen (DE)
(74) Vertreter: Winter, Brandl - Partnerschaft mbB

(57) **Zusammenfassung**

Offenbart ist eine Hülse (2) für einen Körper (6) einer Implantatschraube (1) insbesondere für die Wirbelsäulenchirurgie und eine Implantatschraube (1) mit einem Körper/Tulpe (6), auf den die Hülse (2) aufgesteckt werden kann und zum Beispiel per Schnappverbindung gesichert werden kann. Die Hülse (2) dient als definierte Anlage (16, 20) für ein Kompressionsinstrument oder für ein Distraktionsinstrument bzw. die Hülse (2) hat mindestens eine derartige Anlage (16, 20). Es können gemäß einem ersten Prinzip vier nasenförmige Anlagen (16) und entlang einer Mittelachse beabstandet weitere Anlagen (20) vorgesehen sein. Oder die Hülse (2) hat gemäß einem zweiten Prinzip schräge z.B. abgeplattete Bereiche (19), so dass sich Freiräume am Außenumfang der Hülse (2) ergeben, die es ermöglichen, dass die Zange (12) sich unter Schrägstellung an die Mittelachse (11) der Hülse (2) und des Körpers (6) annähern kann.

## Beschreibung

### Technisches Gebiet

Die vorliegende Offenbarung betrifft eine (Montage-)Hülse zum Aufsetzen auf ein als Körper / Tulpe bezeichnetes (schwenkbares) Anschlussteil einer polyaxialen Implantatschraube/ Pedikelschraube insbesondere für ein Wirbelsäulen-Stabilisierungssystem. Insbesondere betrifft die vorliegende Offenbarung eine (hülsenförmige) Montagehilfe, welche nach einem unabhängigen Aspekt der Offenbarung dafür vorgesehen und ausgebildet ist, beispielsweise während einer offenen oder minimalinvasiven OP auf die Tulpe einer Pedikelschraube (dreh- und/oder kippfest) aufgesetzt/ aufgeschnappt/ aufgedreht zu werden, um damit die Tulpe (bezüglich des Pedikelschraubenschafts) leichter (manuell) ausrichten zu können und das sichere Einbringen/ Einschrauben einer Madenschraube (Setscrew) in die Tulpe zu deren Lagefixierung und Arretierung eines in die Tulpe bereits eingelegten Verbindungs-/Koppelstabs (relativ zum Pedikelschraubenschaft) zu gewährleisten.

Des Weiteren ist die (Montage-) Hülse bzw. die (hülsenförmige) Montagehilfe nach einem anderen unabhängigen Aspekt der Offenbarung dazu vorgesehen und ausgebildet, dass sich die Tulpe insbesondere bei Distraktions- und/oder Kompressionsmanövern zweier benachbarter Wirbel (K/D) mittels eines Distraktions- und/oder Kompressionsinstruments relativ zur Pedikelschraube (selbsttätig schwenkend) ausrichtet, wofür die Montagehilfe mit wenigstens einem definierten Anlagepunkt / Anlagebereich für das Distraktions- und/oder Kompressionsinstrument versehen oder ausgebildet ist, der so an der Montagehilfe platziert ist, dass durch den dortigen Eintrag einer (Radial-)Kraft, beispielsweise einer Distraktions- oder Kompressionskraft in die hülsenförmige Montagehilfe ein Verschwenken/ Verkippen der Tulpe (zusammen mit der Montagehilfe) mit Bezug zum Schraubenschaft erzeugt (erzwungen) wird.

### Hintergrund der Erfindung

Wirbelsäulen-Stabilisierungssysteme dienen prinzipiell zur dorsalen Stabilisierung der Wirbelsäule beispielsweise bei Frakturen, Tumoren, Entzündungen, Deformitäten und degenerativ bedingten Instabilitäten mittels transpedikulärer Verschraubungen. Dabei werden z.B. Pedikelschrauben jeweils in den Pedikeln benachbarter Wirbel platziert (eingeschraubt), worauf eine winkelstabile Verbindung zwischen den Pedikelschrauben zweier benachbarter Wirbel über einen sich entlang der Wirbelsäule erstreckenden Koppelstab geschaffen wird. Die Pedikelschrauben und der Koppelstab bilden somit ein Wirbel-Stabilisierungssystem (starres Gerüst). Implantatschrauben für ein Wirbelsäulen-Stabilisierungssystem können auch im Sakrum- oder Iliumbereich eingesetzt werden.

Pedikelschrauben werden von einem Operateur in den Pedikelkanal eines Wirbels eingesetzt und durch Verschrauben verankert. Dabei richtet der Operateur die Pedikelschrauben auf Basis der Orientierung des Pedikelkanals Patienten-individuell aus. Wenn die Pedikelschrauben gesetzt sind, wird der vorstehend genannte Koppelstab der richtigen Länge ausgewählt und ggf. in seiner Krümmung an die Pedikelschrauben sowie deren jeweilige Position angepasst. Da die Pedikelschrauben jedoch in Abhängigkeit des jeweiligen Pedikelkanals unterschiedlich ausgerichtet sind, haben diese in der Regel eine schwenk- und/oder drehbar am Kopf der Pedikelschraube gelagerte Tulpe (längsgeschlitzte Aufnahmehülse), in welche der Koppelstab eingelegt wird, wobei die Tulpe schwenkend und/oder drehend ausgerichtet werden kann.

### Stand der Technik

Grundsätzlich werden monoaxiale und polyaxiale Implantatschrauben (Pedikelschrauben) unterschieden. Im Fall einer monoaxialen Implantatschraube sind der Außengewindeschaft (Schraube) und die Tulpe (nachfolgend auch als Körper bezeichnet) fest miteinander verbunden oder (stoff-) einstückig miteinander ausgebildet, sodass die Tulpe eine bezüglich der Schraube feste (nicht veränderbare) Ausrichtung hat. Diese monoaxialen Implantatschrauben haben jedoch den Nachteil, dass ein, zwei benachbarte Wirbel miteinander verbindender Koppelstab nur schwer in die bezüglich der Schrauben starren Tulpen bzw. Körper der zu koppelnden Implantatschrauben eingeführt und stabil darin gehalten werden kann.

Die von der Erfindung betroffene polyaxiale Implantatschraube hat hingegen an ihrem Außengewindeschaft / Pedikelschraubenschaft einen zumeist kugelförmigen oder (semi-) sphärischen Gelenkkopf, der von dem, als separates Bauteil gefertigten (hohlzylindrischen) Körper (Body) oder Tulpe relativ-verschwenkbar umgriffen und im Übergangsbereich zwischen Schaftkopf und Außengewindeschaft hintergriffen wird. Damit ist zwischen dem Außengewindeschaft und dem Körper/ der Tulpe ein Kugel- oder Scharniergelenk gebildet. Der Körper/ die Tulpe hat ferner einen U-förmigen Längsschlitz, in den der Koppelstab quer zur Tulpen-Mittelachse eingeführt werden kann. Durch das Gelenk zwischen Pedikelschraubenschaft und Tulpe kann der Körper / die Tulpe nach Versenken des Außengewindeschafts z.B. im Pedikelkanal eines Wirbels oder in einem Ilium oder Sakrum relativ zum Schaftkopf verschwenkt und/oder verdreht werden, um eine gewünschte Lage und Ausrichtung des U-förmigen Längsschlitzes im Wesentlichen unabhängig zur Ausrichtung des Außengewindeschafts zu erhalten. Die Hinterschneidung an der Tulpe verhindert dabei, dass der Körper / die Tulpe und damit der daran gehaltene bzw. in den Längsschlitz eingesetzte Koppelstab vom Schaftkopf (axial) abgezogen werden kann. Schließlich weist die Tulpe innenseitig ein Innengewinde auf, in das eine Madenschraube eingedreht wird, die sich gegen den eingesetzten Koppelstab anlegt.

Derartige polyaxiale Implantatschrauben erleichtern es dem Operateur, den Koppelstab in den Körper einzusetzen/einzuführen/zu implantieren. Ist der Operateur mit der Lage des Koppelstabs und des Körpers zufrieden, wird der Körper mittels der Setscrew / Madenschraube bei zwischengelagertem Koppelstab als Schraubkraft-Übertragungselement (Ein-Schrauben-Prinzip) oder durch eine zusätzliche Schraube/Schraubenmutter zwischen Körper und Koppelstab (Mehr-Schrauben-Prinzip) am Schaftkopf lagefixiert.

In der auf die Anmelderin zurückgehenden DE 10 2016 108 504 A1 ist eine polyaxiale Pedikelschraube offenbart, deren gegenüber dem Außengewindeschaft schwenkbarer Körper / Tulpe zwei diametral sich gegenüberliegende, axial sich erstreckende Körperabschnitte (oder Flanken, oder Tulpenblätter) hat, die zwischen sich den U-förmigen Längsschlitz ausbilden, in den der Koppelstab eingesetzt wird. An der Innenseite des (hohlzylindrischen) Körpers bzw. an der Innenseite der sich gegenüberliegenden Flanken ist ein Innengewinde gebildet, in das die Setscrew eingeschraubt wird, nachdem der Koppelstab eingesetzt und zusammen mit dem Körper positioniert wurde. Genauer gesagt ist die Setscrew als Madenschaube ausgebildet, die mittels zweier Teilgewinde der beiden Körperabschnitte in den Körper eingeschraubt wird.

Das Setzen der Setscrew birgt indessen das Risiko, dass sie schräg angesetzt wird und verkantet, was im besten Fall Zeit kostet und im schlimmsten Fall das Innengewinde im Körper/in der Tulpe beschädigt. Im letzteren Fall muss die gesamte Implantatschraube ausgetauscht werden.

Aus dem Stand der Technik ist es daher bereits bekannt, eine offene oder minimalinvasive Operation mit (Montage-)Hülsen durchzuführen, die auf die Körper / Tulpen in axialer Verlängerung jeweils gesetzt werden. Derartige (Montage-)Hülsen dienen zum einfacheren Ausrichten der Körper und können das Setzen/Einschrauben der Setscrew erleichtern, da sie bereits die Schrauben-Achse vorgeben und damit die Setscrew außenseitig führen. Die Hülsen können auch als axialen Gegenhalter dienen, indem sie sich mit dem Körper / der Tulpe zumindest in Axialrichtung provisorisch verrasten und gleichzeitig über einen internen Schraubmechanismus (interne Vorspannschraube) eine provisorische/temporäre Verspannkraft zwischen dem Schaftkopf und der Tulpe erzeugen, wodurch der Körper / die Tulpe provisorisch/vorläufig am Schaftkopf (vor-)fixiert werden kann. Auch können die Montagehülsen den Körper während Kompressions- oder Distraktionsmanövern über/mit entsprechende(n) Zangen stützen, erlauben jedoch keine oder nur eingeschränkte Winkelung der Tulpe zur Zange.

Nachteilig ist zudem, dass die Montagehülsen des Standes der Technik aufgrund des internen Schraubmechanismus eine bestimmte Baugröße aufweisen müssen und daher die Sicht auf die Implantatschrauben versperren/einschränken und den Arbeitsbereich, z.B. beim Setzen eines Cage zwischen zwei Wirbeln, einengen.

Bei der Distraktion und Kompression der Körper / Tulpen zweier benachbarter Implantatschrauben bzw. von Implantatschrauben zweier benachbarter Wirbel mit Zangen ohne zusätzlicher Montagehilfen können also Situationen entstehen, in welchen sich der Körper/ die Tulpe nicht mehr richtig an den Koppelstab (fest) anlegen kann bzw. relativ zum Koppelstab derart schräg steht, dass die zur Korrektur der fehlerhaften Ausrichtung der Tulpe benötigten Kräfte nicht von der Setscrew aufgebracht werden können. Bei einer falschen Ausrichtung der Tulpe bezüglich des Koppelstabs besteht auch die Möglichkeit, dass die Setscrew während des Festziehens ein Kippmoment erfährt. In anderen Worten ausgedrückt kann sich die Ausrichtung (Winkelstellung) der Tulpe zum bereits eingelegten Koppelstab beim Vorgang einer Distraktion oder Kompression zweier benachbarter Wirbel verändern. Wird in diesem Zustand die Madenschraube festgezogen, drückt diese ggf. in einem schrägen Winkel (also außermittig) gegen den Koppelstab. Dadurch wird das Innengewinde in der Tulpe einseitig belastet. Dies kann zu einer reduzierten Verriegelungskraft oder gar zum Versagen des Gewindes zwischen Setscrew und Körper oder zu einer Deformation der Körperabschnitte / Flanken des Körpers und einem Herausspringen der Setscrew aus dem Innengewinde führen.

### Kurzbeschreibung der Erfindung

Aufgabe der vorliegenden Offenbarung ist es, eine Montagehülse bzw. hülsenförmige Montagehilfe für einer Implantatschraube insbesondere eines Wirbelsäulen-Stabilisierungssystems, zu schaffen, die während der Operation übergangsweise auf den Körper (Tulpe) der Implantatschraube aufgesetzt wird / aufsetzbar ist, und die insbesondere bei Distraktions- und Kompressionsvorgängen (Auseinanderdrücken oder Zusammenziehen zweier benachbarter Wirbel) mittels entsprechender Zangen gleichzeitig ein (verbessertes) Ausrichten des Körpers (Tulpe) bezüglich eines bereits eingelegten Koppelstabs bewirkt. Ferner ist es eine andere bevorzugte Aufgabe der vorliegenden Offenbarung, eine Montagehülse bzw. hülsenförmige Montagehilfe für einer Implantatschraube insbesondere eines Wirbelsäulen-Stabilisierungssystems, zu schaffen, die leicht und schnell am Körper (Tulpe) der (polyaxialen) Implantatschraube montiert werden kann.

Diese Aufgabe wird gelöst durch eine Montagehülse bzw. hülsenförmige Montagehilfe mit der Merkmalskombination des Anspruchs 1 und/oder durch eine Anordnung/ein Implantatschraubenset mit einer derartigen Montagehülse und mit einer Implantatschraube, insbesondere für ein oder eines Wirbelsäulen-Stabilisierungssystem(s), mit der Merkmalskombination des Anspruchs 14.

Die Montagehülse gemäß der vorliegenden Offenbarung ist nach einem (unabhängig beanspruchbaren) Aspekt der vorliegenden Offenbarung zum (stirnseitigen) Aufsetzen oder Aufstecken und zum Befestigen per axialem Hinterschnitt, z.B. per Aufschnappen oder auch per Bajonettverschluss auf einen schwenkbaren Körper (Tulpe) einer polyaxialen Implantatschraube ausgebildet und eingerichtet. Dazu hat die Montagehülse einen (axial mittig angeordneten) Verrast- oder Kopplungsabschnitt zur (schnell-)lösbaren Kopplung der Montagehülse an dem Körper/ Tulpe z.B. in Form einer (hülsenseitigen) Schnapp- oder Clickvorrichtung, die zur lösbaren Fixierung oder Verrastung der Hülse am Köper eingerichtet und ausgelegt ist. Die offenbarungsgemäße Montagehülse bietet gegenüber den Hülsen des vorstehend genannten Standes der Technik somit den Vorteil, dass sie schnell und einfach (werkzeuglos sowie selbsttätig verrastend) auf den Körper aufgesetzt und dort fixiert werden kann und dabei wenig Raum für den Kopplungsabschnitt benötigt. Dadurch verbleibt mehr Raum für weitere Operationsvorgänge, wie z.B. dem Setzten eines Cages zwischen zwei benachbarte Wirbel.

Die Montagehülse ist dazu ausgelegt, dass sie bis zur Lösung/Freigabe der Verbindung bzw. der Verrastung oder Kopplung (z.B. durch Betätigung oder Bewegung der hülsenseitigen Schnapp- oder Clickvorrichtung) (axial-)fest auf dem Köper (Tulpe) sitzt und dabei auch größere Hebelkräfte wie insbesondere die auf die Tulpe bzw. die Montagehülse einwirkenden Radialkräfte eines Distraktions- oder Kompressionsinstruments ohne unerwünschte Relativbewegung der Montagehülse zum Körper auf den Körper übertragen kann.

Gemäß einem anderen ggf. unabhängig beanspruchbaren Aspekt der Offenbarung ist an einer Hülsenmantel-Außenseite der Montagehülse ein vordefinierter Anlagebereich (Anlageerhebung), vorzugsweise in Form von mindestens einem Paar (zwei) Anlagenvorsprünge oder Anschläge z.B für ein Distraktionsinstrument und/oder für ein Kompressionsinstrument ausgebildet oder angeordnet. In anderen Worten ausgedrückt hat die Montagehülse einen vorbestimmten, sich überschneidenden Axial- und Umfangsbereich (entspricht in seiner Gesamtheit dem Anlagebereich), der in Radialrichtung über den (übrigen) Hülsenmantel-Außenumfang (an (allen) anderen (benachbarten) Bereichen der Montagehülse) nach außen absteht/vorragt. Die Umfangsposition des Anlagebereichs ist an der Montagehülse vorzugsweise so gewählt, dass der Anlagebereich (in angesetztem Zustand der Montagehülse auf der Tulpe) in Längsrichtung des eingelegten oder einzulegenden Koppelstabs (d.h. in Koppelstab-Einlegrichtung) weist. Des Weiteren ist die Axialposition des Anlagebereichs an der Montagehülse vorzugsweise so gewählt, dass sich der Anlagebereich in angesetztem (montiertem) Zustand der Montagehülse auf der Tulpe in einem dem Schraubenkopf zugewandten (distalen) Axialabschnitt der Tulpe (möglichst nahe am Schraubenkopf der Implantatschraube, vorzugsweise im unteren/distalen axialen Viertel der Montagehülse) befindet und weiter bevorzugt axial zwischen dem Schraubenkopf und dem Koppelstab liegt.

Die Anlagen können gemäß einem ersten Prinzip jeweils als ein von der Hülsenmantel-Außenseite radial nach außen hervorstehender Vorsprung (z.B. Nase oder Leiste) gebildet sein und/oder die Anlagen sind gemäß einem zweiten Prinzip einfach von einem ersten axialen Rand (dem Schraubenkopf zugewandte axiale Stirnkante) der Montagehülse gebildet, wohingegen die Hülsenmantel-Außenseite sich ausgehend von dem ersten axialen Rand entlang einer Mittelachse der Montagehülse hin zu einem zweiten axialen Rand der Montagehülse (vom Schraubenkopf abgewandte axiale Stirnkante) verjüngt (Kegel- oder Pyramidenform). Selbstverständlich sind auch beide Prinzipien in Kombination realisierbar.

Wird bei angesetzter Montagehülse beispielsweise im Rahmen einer Distraktion ein Distraktionsinstrument an die eine Implantatschraube (z.B. Pedikelschraube) angelegt, kommt das Distraktionsinstrument aufgrund der wie vorstehend definierten Grundform der Montagehülse zunächst mit dem (radial vorragenden) Anlagebereich der Montagehülse in Kontakt und leitet dort eine Radialkraft (Distraktionskraft) in die Montagehülse ein. Diese Kraft wird über die Tulpe und den Implantatschraubenschaft in den zugehörigen Wirbel übertragen, der demzufolge von seinem benachbarten Wirbel beabstandet wird. Dabei erfährt die Tulpe in Abhängigkeit ihrer ursprünglichen Ausrichtung (automatisch/selbsttätig) eine Kippbewegung um den Schraubenkopf hin zum Distraktionsinstrument, wodurch sie sich im Wesentlichen senkrecht zum bereits eingelegten Koppelstab ausrichtet. Jetzt kann die Madenschraube festgezogen und damit die Lage von Tulpe und Koppelstab fixiert werden, ohne dass das Innengewinde der Tulpe zu einem Versagen gebracht wird.

Die vorstehend genannte Kippbewegung der Tulpe hin zum Distraktionsinstrument resultiert dabei quasi zwangsläufig aus der Translationsbewegung des zugehörigen Wirbels. D.h. wird der eine Wirbel von seinem benachbarten Wirbel längs des bereits eingelegten Koppelstabs mittel des Distraktionsinstruments wegbewegt, kann sich die Tulpe um den Schraubenkopf verschwenken, bzw. zum Koppelstab ausrichten, da die Tulpe bzw. die Montagehülse infolge des offenbarungsgemäß ausgebildeten Anlagebereichs (punkt- oder linienförmiger Radialvorsprung) nicht großflächig an das Distraktionsinstrument anliegt.

Je weiter (beim ersten Prinzip) die Vorsprünge (z.B. Nasen) radial abstehen bzw. je stärker (beim zweiten Prinzip) die Verjüngung ist, desto weiter kann die Montagehülse und damit der Körper (Tulpe) hin zu dem Distraktions- oder Kompressionsinstrument um den Schraubenkopf verschwenken.

Im Falle der als Vorsprünge gebildeten Anlagen (erstes Prinzip) sind die beiden Anlagen jedes Paares vorzugsweise in einem solchen Umfangsabstand zueinander angeordnet und/oder stehen derart weit von der Hülsenmantel-Außenseite hervor, dass eine die radial äußersten Stellen der beiden Anlagen verbindende, gedachte Gerade die Hülsenmantel-Außenseite nicht schneidet. Außerdem hat die Montagehülse bevorzugt zwei sich diametral gegenüberliegende Axialschlitze (in Übereinstimmung mit den Axialschlitzen in der Tulpe zur Aufnahme des Koppelstabs), wobei die beiden Anlagen eines Paares in Umfangsrichtung gesehen beidseits des einen Axialschlitzes (also insgesamt zwei Anlagen) oder beidseits beider Axialschlitze (also insgesamt vier Anlagen) platziert sind.

Im Falle der Verjüngung der Hülsenmantel-Außenseite (zweites Prinzip) ergeben sich die vorstehend genannten vier Anlagen beidseits beider Axialschlitze am Umfangsrand mit dem größten Radius quasi zwangsläufig von selbst. Dabei kann die Montagehülse die Form eines Huts oder einer Kappe annehmen.

Insbesondere im Fall einer Kombination aus den beiden vorstehend beschriebenen Prinzipien kann die Verjüngung der Montagehülse auch nur in einem Umfangsbereich axial unmittelbar oberhalb der beiden (nasenförmigen) Anlagevorsprünge ausgebildet sein, wohingegen die übrigen Umfangsbereiche der Montagehülse davon abweichen, z.B. zylinderförmig sein können. Auch kann die Verjüngung bzw. der sich verjüngende Umfangsabschnitt kegelartig (also umfangsgekrümmt) oder pyramidenartig (also plan) sein.

Die Montagehülse hat üblicherweise den vorstehend bereits genannten ersten umlaufenden stirnseitigen Rand, der dafür vorgesehen ist, der Implantatschraube oder ihrem Außengewindeschaft zugewandt zu sein. Durch diesen ersten Rand hindurch wird der Körper (Tulpe) zumindest abschnittsweise in die Montagehülse eingeführt, wenn die Montagehülse auf den Körper aufgesetzt wird. Demzufolge sind an diesem ersten Rand die zwei bezüglich der Mittelachse einander diametral gegenüberliegenden, vorzugsweise U-förmige Koppelstabausnehmungen oder Ausbuchtungen / Axialschlitze zur Durchführung oder Aufnahme des Koppelstabs in Querrichtung zur Mittelachse ausgebildet, welche sich ausgehend vom ersten Rand axial erstrecken. Je weiter diese axiale Erstreckung ist, also je tiefer diese Koppelstabausnehmungen oder Ausbuchtungen sind, desto weiter kann der Körper (Tulpe) in die Montagehülse axial eintauchen, und desto weiter kann sich der erste Rand an den Außengewindeschaft bzw. an den Schraubenkopf der Implantatschraube annähern, wenn die Montagehülse auf den Körper (Tulpe) gesetzt wird.

Damit können die Anlagen/Anlagebereiche der Montagehülse weiter in Richtung hin zum Schraubenkopf positioniert bzw. genauer gesagt in der Nähe des Schaftkopfs angeordnet werden. Dies bringt Vorteile bei den Kompressions- und Distraktionsmanövern, da das, aus der Radialkrafteinleitung resultierende, Kippmoment der Montagehülse und des damit fest verbundenen Körpers gegenüber dem Außengewindeschaft vermindert wird.

Insbesondere wenn das mindestens eine Paar Anlagen an dem ersten Rand der Montagehülse angeordnet oder gebildet ist, können die Anlagen in der Nähe des Außengewindeschaftes positioniert bzw. genauer gesagt in der Nähe des Implantatschraubenkopfs angeordnet werden. Dies bringt Vorteile bei den Kompressions- und Distraktionsmanövern, da das Kippmoment der Montagehülse und des damit fest verbundenen Körpers gegenüber dem Außengewindeschaft vermindert wird.

Vorzugsweise sind an diametral einander gegenüberliegenden Umfangsabschnitten der Montagehülse jeweils ein Paar Anlagen oder ein Paar Anschläge vorgesehen, wie dies vorstehend bereits angedeutet wurde. Dann kann die Montagehülse während der Operation einerseits von dem Distraktionsinstrument und andererseits von dem Kompressionsinstrument beaufschlagt werden. Diese Weiterbildung wird in dieser Schrift gedoppelte Montagehülse genannt.

Bei einem Ausführungsbeispiel der gedoppelten Montagehülse ist an dem ersten Rand in Umfangsrichtung gesehen beidseitig der beiden Koppelstabausnehmungen (Axialschlitze) oder Ausbuchtungen jeweils eine Anlage vorgesehen. Die beiden Anlagen jedes Paares begrenzen vorzugsweise die betreffende Koppelstabausnehmung oder Ausbuchtung beidseitig. Dadurch sind die Kräfte an der Montagehülse während der Distraktions- und/oder Kompressionsmanöver mit den entsprechenden Zangen vergleichmäßigt.

Bei einem anderen Ausführungsbeispiel der gedoppelten Montagehülse sind die Anlagen der beiden Anlagenpaare und folglich auch der erste Rand entlang der Mittelachse betrachtet näher an dem Schraubenkopf angeordnet, als der Koppelstab. Damit ist das Kippmoment der Montagehülse und des damit fest verbundenen Körpers (Tulpe) gegenüber dem Außengewindeschaft minimiert.

Bei dem Ausführungsbeispiel der gedoppelten Montagehülse mit Verjüngung der Hülsenmantel-Außenseite (zweites Prinzip) ist diese Verjüngung dadurch gebildet, dass die Hülsenmantel-Außenseite eine kegelform oder kegelstumpfform aufweist. Oder es sind an zwei bezüglich der Mittelachse einander diametral gegenüberliegenden Bereichen der Hülsenmantel-Außenseite Flachseiten oder axial sich erstreckende Außenschrägen vorgesehen oder ausgebildet, die vorzugsweise in einem Längsschnitt der Montagehülse betrachtet eine Trapezform ergeben.

Die Flachseiten oder Außenschrägen (zweites Prinzip) können sich ausgehend von dem ersten Rand und/oder ausgehend von dem jeweiligen Paar Anlagen erstrecken.

Bei dem Ausführungsbeispiel der gedoppelten Montagehülse erstrecken sich die Flachseiten oder Außenschrägen bis zu einem zweiten stirnseitigen Rand, der dem ersten Rand axial gegenüberliegt, wobei die Flachseiten oder Außenschrägen in Richtung hin zum zweiten stirnseitigen Rand aufeinander zulaufen und dadurch die Verjüngung bilden.

Weiter vorzugsweise ist jede Anlage / die Anlageanordnung (das Widerlager) in Abhängigkeit der verwendeten Zange(n) geformt und bildet beim ersten Prinzip einen radial nach außen sich erstreckenden, vorzugsweise nasenförmigen Vorsprung, der im Bereich der Tulpen-zugewandten Stirnseite der Hülse am weitesten radial vorragt und einen kontinuierlich zur Hülsenaußenseite zurücklaufenden axialen Nasenrücken aufweist.

Bei der einen, besonders bevorzugten Ausgestaltung sind an bezüglich einer Mittelachse einander gegenüberliegenden Bereichen der Hülse jeweils ein Paar Anlagen vorgesehen (gedoppelte Montagehülse). Wenn diese Anlagenpaare an den beiden einander diametral gegenüberliegenden Umfangsbereichen der Montagehülse gleich sind, bietet die Hülse in zwei verschiedene um 180 Grad zueinander gedrehte Positionen den Vorteil der auf gleiche Weise verminderten Abrutschgefahr. Wenn diese beiden Anlagenpaare oder Anlageanordnungen an den beiden einander diametral gegenüberliegenden Bereichen der Hülse jedoch ungleich sind, kann der eine Umfangsbereich der Hülse für ein Distraktionsinstrument ausgelegt und optimiert sein, während der andere Umfangsbereich für ein Kompressionsinstrument ausgelegt und optimiert sein kann.

Vorzugsweise ist mindestens eine Anlage an einem um eine Mittelachse der Hülse umlaufenden ersten Rand der Hülse vorgesehen oder gebildet, der der Implantatschraube oder ihrem Außengewindeschaft zugewandt ist.

Bei einem Ausführungsbeispiel der Montagehülse sind an dem ersten Rand die zwei bezüglich der Mittelachse einander diametral gegenüberliegenden (vorzugsweise bogenförmige z.B. halbkreisförmige) bereits genannten Koppelstabausnehmungen vorgesehen. In diesem Fall kann die Hülse entlang bzw. in Richtung der Mittelachse vergleichsweise weit auf den Körper aufgesteckt/aufgeschoben werden, so dass z.B. ein sicherer Halt entsteht. Weiterhin wird dadurch ermöglicht, dass die Anlagen weit in Richtung zum Implantatschraubenkopf bewegt bzw. schließlich in der Nähe des Schraubenkopfs positioniert und fixiert werden können. Durch dieses Aufstecken bzw. Aufschieben der Hülse auf den Körper/ Tulpe ist insbesondere eine großflächige Stützanlage zwischen Außenwandungen von Tulpenabschnitten und inneren Stützwandungen der Hülse möglich, um die Körperabschnitte mechanisch zu stützten.

Wenn beim zweiten Prinzip an zwei bezüglich der Mittelachse einander gegenüberliegenden Bereichen der Außenseite des Hülsenmantels konische Außenwandabschnitte oder Außenschrägen vorgesehen sind, die sich bis zu einem zweiten Rand erstrecken, der dem ersten Rand axial gegenüberliegt, kann die Hülse eine kappenartige oder hutartige Form aufweisen. Damit kann die Hülse auch als Cap bezeichnet werden. In anderen Worten ausgedrückt kann die Außenseite oder der Hülsenmantel trapezförmig aufeinander zulaufende Flachseiten aufweisen. Damit ist ein Abwinkeln der Hülse und damit des Körpers/ Tulpe zum Koppelstab und / oder zur Zange erleichtert. Dazu kann sich der Körper/ Tulpe mit der Hülse neigen, wobei sich der benachbarte konische Außenwandabschnitt bzw. die benachbarte Außenschräge bzw. die benachbarte Flachseite der entsprechenden Zange annähert und sogar in Anlage damit geraten kann.

In anderen Worten hat die Hülse beim zweiten Prinzip vorzugsweise schräge und vorzugsweise abgeplattete Bereiche, so dass sich Freiräume am Außenumfang der Hülse ergeben, die es ermöglichen, dass die Zange sich unter Schrägstellung an die Mittelachse der Hülse und des Körpers/ Tulpe annähern kann und in einem Grenzfall in Kontakt mit allen Anlagen der Anlageanordnung kommt.

Um das Verschwenken zwischen der Zange und der Montagehülse definiert zu begrenzen ist es besonders bevorzugt, wenn jeweils im Bereich des zweiten (kleindurchmessrigen) Rands (gegenüber dem ersten, großdurchmessrigen Rand) zumindest ein weiteres Paar Anlagen für die Zange ausgebildet oder angeordnet sind. Damit können zumindest zwei einander axial gegenüberliegende Anlageanordnungen geschaffen sein, die jeweils ein oder zwei Paare Anlagen aufweisen.

An einem den ersten Rand gegenüberliegende und/oder von der Außengewindeschaft abgewandten Bereich der Hülse kann eine axial sich erstreckende Instrumentenöffnung zur Durchführung eines Schraubendrehers für eine Setscrew (Setscrewdriver) und/oder eines Gegenhalters vorgesehen sein. Die Instrumentenöffnung kann von dem zweiten Rand umfasst sein.

An dem zweiten Rand der Montagehülse, der auch als von der Implantatschraube bzw. deren Außengewindeschaft abgewandter Rand bezeichnet werden kann, sind vorzugsweise konische (trichterartige) Einführabschnitte oder (innenseitige) Einführschrägen für die Setscrew gebildet. Alternativ oder in Ergänzung können an einer Innenseite der Montagehülse Führungsflächen vorzugsweise als Kreiszylinderteilflächen für die Setscrew gebildet sein. Beide Ausgestaltungen erleichtern bzw. erzwingen ein coaxiales Einführen der Setscrew entlang der Mittelachse der Montagehülse, die mit der Mittelachse eines Innengewindes des Körpers/Tulpe für die Setscrew zusammenfällt, wenn die Montagehülse auf die Tulpe aufgesetzt ist.

Eine hülsenseitige Schnappvorrichtung ist bei einer fertigungstechnisch einfachen Ausgestaltung von mindestens einer Federlasche/Federzunge (vorzugsweise von zwei einander gegenüberliegenden Federlaschen) gebildet. Die eine oder die beiden Federlaschen erstreckt/erstrecken sich bei einer konkreten Ausgestaltung axial von dem zweiten Rand (Federzungenwurzel) in Richtung zum ersten Rand (freie Federzungenspitze).

Die Federlasche(n) kann/können platzsparend in einer (jeweiligen) Durchgangsausnehmung der Montagehülse angeordnet sein, die sich dann vorzugsweise zusammen mit der Federlasche vom zweiten Rand in Richtung zum ersten Rand erstreckt.

An einem freien Endabschnitt der mindestens einen Federlasche ist vorzugsweise eine Anlage-/Rasteingriffsfläche zum Hintergreifen des Körpers/Tulpe angeordnet oder gebildet. Zwischen dem zweiten Rand und der Anlagefläche kann ein Federlaschen-Betätigungsabschnitt (an der Federlasche) beispielsweise in Form von radial nach innen ragenden Vorsprüngen angeordnet oder gebildet sein, über den die Federlasche beispielsweise durch axiales Einführen eines Spreizkeils in die Hülse nach außen weg von der Mittelachse gedrängt werden kann, um so die freigegebene Montagehülse vom Körper wieder zu lösen.

Zum Lösen der Schnappverbindung der Montagehülse vom Körper kann alternativ an der Innenseite der Hülse eine Nut vorgesehen sein, die sich in Umfangsrichtung der Hülse erstreckt, und die in der Durchgangsausnehmung mündet. Dabei mündet die Nut in die Durchgangsausnehmung entlang einer Mittelachse der Hülse betrachtet in derjenigen axialen Position oder Stelle, die der des Betätigungsabschnitts entspricht. Dann kann der Gegenhalter gedreht werden, wobei seine mindestens eine am Außenumfang angeordnete Nase durch jeweils eine Nut bewegt wird und schließlich von innen gegen den Betätigungsabschnitt drückt. Dadurch wird die Federlasche nach außen gedrängt, und so die Hülse vom Körper gelöst oder entriegelt.

Bei einer fertigungstechnisch und anwendungstechnisch einfachen Ausgestaltung ist die Hülse rotationssymmetrisch zur Mittelachse und/oder spiegelsymmetrisch zu einer Mittelebene.

Insbesondere im Falle der oben genannten Symmetrie können an einander gegenüberliegenden Seiten der Hülse zwei Koppelstabausnehmungen für den Koppelstab und/oder zwei Anlagenpaare und/oder zwei konische Außenwandabschnitte bzw. Außenschrägen vorgesehen sein.

Insbesondere im Falle der oben genannten Symmetrie können an einander gegenüberliegenden Seiten der Hülse zwei Federlaschen (vorzugsweise mit jeweiligen Durchgangausnehmungen) vorgesehen sein.

Insbesondere im Falle der oben genannten Symmetrie können an einander gegenüberliegenden Innenbereichen der Hülse zwei Nuten vorgesehen sein, in die sich der Gegenhalter mit zwei einander gegenüberliegende Nasen bajonettartig eindrehen lässt, um die Hülse vom Körper zu lösen.

Die Anordnung/Implantatschraubenset gemäß der Offenbarung hat wenigstens eine Implantatschraube mit einem gegenüber einem Außengewindeschaft schwenkbaren Körper (Tulpe). Der Körper hat zwei einander gegenüberliegende Körperabschnitte (Tulpenblätter), zwischen denen eine Koppelstab-Einlegeöffnung zur Aufnahme eines Koppelstabs vorgesehen ist. Weiterhin hat die Anordnung/Implantatschraubenset wenigstens eine vorbeschriebene Montagehülse, die (vorzugsweise mittels ihrer Schnappvorrichtung) an dem Körper/Tulpe übergangsweise bzw. lösbar befestigbar ist, und die die beiden Körperabschnitte (Tulpenblätter) zumindest abschnittsweise außenseitig umgreift. Die Auslegung der Schnapp- oder Clickvorrichtung und die Formgebung des Körpers und der Montagehülse sind vorzugsweise derart aufeinander abgestimmt, dass die Fixierung der Hülse an dem Körper in allen sechs Raumachsen wirkt.

Da vorzugsweise an der Außenseite der Montagehülse mindestens ein Paar Anlagen für ein medizinisches Instrument, insbesondere für ein Distraktionsinstrument und/oder für ein Kompressionsinstrument ausgebildet oder angeordnet ist, bekommt der Körper übergangsweise für die Zeit der aufgesetzten Hülse insbesondere für die Distraktions- bzw. Kompressionsmanöver eine äußere Form, die dem entsprechenden Zangentyp oder vorzugsweise beiden Zangentypen eine optimierte Anlageanordnung bietet. Diese Anlageanordnung ist im Zusammenwirken mit der Zange metastabil oder stabil oder sogar derart formschlüssig, dass ein Abrutschen der Zange gegenüber dem Körper vermieden ist.

Wenn Außenwandungen der Körper-/Tulpenabschnitte und innere Stützwandungen der Montagehülse teilkreiszylindrisch sind und aneinander anlegbar sind oder aneinander anliegen, ist die Hülse zu einer Stütz- und Stabilisierungshülse weitergebildet. Diese verhindert ein Auseinanderdrängen der beiden Körper-Tulpenabschnitte (Tulpenblätter) nach radial außen, auch wenn die Setscrew gegen den Koppelstab festgezogen/festgeschraubt wird.

Vorzugsweise sind an den einander gegenüberliegenden Außenwandungen der Körperabschnitte (Tulpenblätter) jeweilige Vertiefung gebildet, in die eine jeweilige Federlasche der Schnappvorrichtung der Hülse zumindest abschnittsweise (insbesondere mit ihrem Endabschnitt) einrastbar oder eingerastet ist.

Damit das Aufschieben und das anschließende Einschnappen der Federlasche(n) möglichst einfach erfolgen kann, ist vorzugsweise eine Spannschräge für die zugeordnete Federlasche, insbesondere für deren Endabschnitt, ausgebildet. Die jeweilige Spannschräge ist an der Außenwandung des Körperabschnitts zwischen einer in die Hülse eingetauchten oder eintauchbaren Stirnseite des Körperabschnitts und der Vertiefung des Körperabschnitts gebildet.

Die Vertiefung kann (entlang der Mittelachse des Körpers betrachtet) axial benachbart zur Spannschräge eine körperseitige Anlagefläche aufweisen, die senkrecht zur Mittelachse des Körpers ist.

Dabei kann der Endabschnitt der mindestens einen Federlasche eine innere hülsenseitige Anlagefläche haben, die ebenfalls senkrecht zur Mittelachse der Hülse ist. Damit ist ein Lösen der Hülse vom Körper ohne Instrument (z.B. Nase des Gegenhalters) nicht möglich.

Alternativ hat der Endabschnitt der mindestens einen Federlasche eine hülsenseitige Anlagefläche, die schräg zur Mittelachse der Hülse ist. Dann ist in Abhängigkeit der Schrägstellung dieser Anlagefläche und der Federkraft der Federlasche auch ein Lösen der Hülse vom Körper ohne zusätzliches Instrument/Werkzeug möglich.

Die Spannschräge und die Vertiefung jedes Körperabschnitts können paarweise ausgebildet sein, wodurch an der Außenwandung jedes Körperabschnitts eine so genannte Männchen-Einbuchtung z.B. gemäß der in der Einleitung gewürdigten DE 10 2016 108 504 A1 gebildet sein kann.

Die Mittelachse des Körpers fällt mit der Mittelachse der Hülse zusammen, wenn die Hülse auf den Körper aufgesteckt ist und mittels der Schnappvorrichtung zumindest übergangsweise fixiert ist.

Die Implantatschraube kann eine Pedikelschraube, eine Iliumschraube, eine Sakrumschraube oder eine iliosakrale Schraube sein, d.h. zum Einschrauben in einem Pedikel, im Sakrum- oder Iliumbereich geeignet sein.

### Kurzbeschreibung der Figuren

Figur 1 ist ein Ausschnitt einer Anordnung bestehend aus einer Pedikelschraube und einer Hülse gemäß einem Ausführungsbeispiel der vorliegenden Offenbarung in einer Ansicht;
Figur 2 ist die Pedikelschraube mit der Hülse aus Figur 1 mit einem Koppelstab und mit einer nur grobschematisch dargestellten Zange in einer ersten Relativposition;
Figur 3 ist die Pedikelschraube mit der Hülse mit dem Koppelstab und mit der nur grobschematisch dargestellten Zange aus Figur 2 in einer zweiten Relativposition;
Figur 4 ist die Pedikelschraube mit der Hülse in einer geschnittenen Darstellung;
Figur 5 ist die Pedikelschraube mit der Hülse in der Darstellung aus Figur 4 mit einem Gegenhalter;
Figur 6 ist die Pedikelschraube mit der Hülse aus den vorhergehenden Figuren in einer Ansicht auf eine Instrumentenöffnung;
Figur 7 ist ein Körper der Pedikelschraube mit der Hülse aus den vorhergehenden Figuren in einer Ansicht mit einer Setscrew; und
Figur 8 ist ein Querschnitt durch den Körper und die Hülse aus den vorhergehenden Figuren.

### Beschreibung der Ausführungsbeispiele

Nachstehend wird ein Ausführungsbeispiel der vorliegenden Offenbarung auf der Basis der zugehörigen Figuren beschrieben.

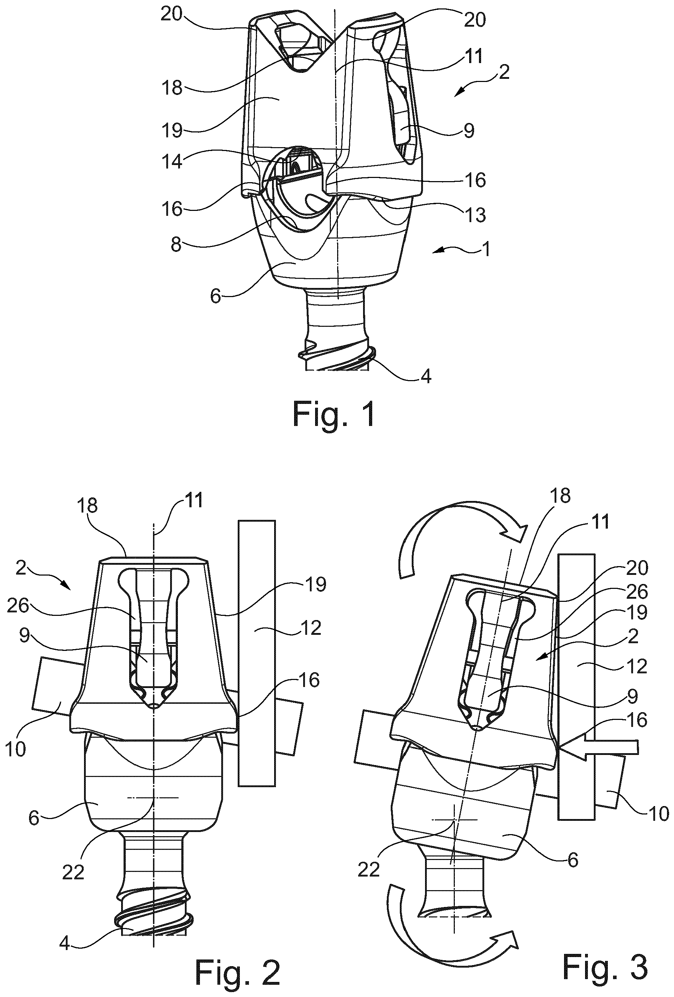

Figur 1 ist ein Ausschnitt einer als Pedikelschraube 1 ausgebildeten Implantatschraube für ein Wirbelsäulen-Stabilisierungssystem mit einer Hülse 2 gemäß einem Ausführungsbeispiel der vorliegenden Offenbarung in einer Ansicht.

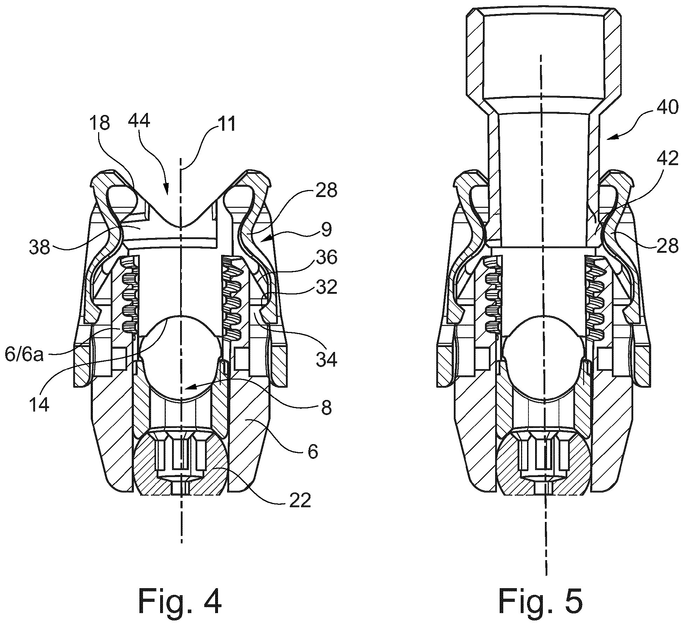

Eine Pedikelschraube 1 hat einen (nur teilweise gezeigten) Außengewindeschaft 4, welcher sich entlang einer (nicht gezeigten) Schraubenlängsachse erstreckt und an dessen gezeigten Ende ein (in den Figuren 4 und 5 gezeigter) Gelenk- oder Schraubenkopf 22 ausgebildet ist, an welchem ein Körper (Tulpe) 6 montiert ist. Der Gelenkkopf 22 bildet mit dem Körper 6 ein Gelenk, sodass der Körper 6 gegenüber dem Außengewindeschaft 4 schwenkbar ist.

Der Körper 6 bildet eine Koppelstab-Einlegeöffnung 8, welche dazu ausgebildet ist, einen quer zu einer Mittelachse 11 des Körpers 6 liegenden (in den Figuren 2 und 3 gezeigten) Koppelstab 10 aufzunehmen und zu fixieren. Dabei erstreckt sich die Stabeinlegeöffnung 8 quer zur Mittelachse 11 durch den Körper 6 hindurch und öffnet sich an beiden einander diametral gegenüberliegenden Seiten des Körpers 6. Im Konkreten ist der Körper 6 hohlzylindrisch mit einer Umfangswand, die an sich diametral gegenüberliegenden Winkelpositionen längsgeschlitzt ist, wodurch sich die vorstehend genannte quer zur Körperlängsrichtung sich erstreckende Einlegeöffnung 8 ergibt.

Die Stabeinlegeöffnung 8 ist zu einer vom Außengewindeschaft 4 abgewandten (in Figur 1 verdeckten) Stirnseite des Körper 6 hin geöffnet, sodass der Koppelstab 10 ausgehend von der einen Stirnseite in die Stabeinlegeöffnung 8 einlegbar ist. In noch anderen Worten ausgedrückt, bildet der Körper 6 in seiner Umfangswand zwei Kerben/Längsschlitze, welche sich bezüglich der Mittelachse 11 diametral gegenüberliegen und sich ausgehend von der, vom Außengewindeschaft 4 abgewandten Stirnseite des Körpers 6 in Richtung hin zu dem Außengewindeschaft 4 erstrecken, um die Stabeinlegeöffnung 8 zu bilden.

Die Montagehülse 2 ist kompatibel zu dieser gezeigten, per se aus dem Stand der Technik bekannten polyaxiale Pedikelschraube 1. Dazu ist die Hülse 2 passgenau auf den schwenkbaren Körper (Tulpe) 6 aufgesetzt bzw. aufsetzbar und mit einer lösbaren Kopplung, vorzugsweise Schnappverbindung (aber auch Bajonettverschluss, Verschraubung, etc.) am Körper 6 gesichert, die im bevorzugten Ausführungsbeispiel von zwei diametral sich gegenüberliegende Federlaschen 9 gebildet ist, von denen in Figur 1 nur eine Federlasche 9 zu sehen ist.

Die Körper 6 und die Hülse 2 haben in dem bestimmungsgemäß aufgesetzten und mit den Federlaschen 9 gesicherten Zustand eine gemeinsame Mittelachse 11.

Die Hülse 2 hat an ihrem, dem Außengewindeschaft 4 zugewandten ersten (stirnseitigen) Rand 13 zwei bogenförmige, vorzugsweise halbkreisförmige Koppelstabausnehmungen (Längsschlitze) 14, von denen in Figur 1 nur eine Koppelstabausnehmung 14 dargestellt ist.

Am ersten Rand 13 sind beidseitig jeder Koppelstabausnehmung 14 jeweiligs eine als Nase ausgestaltete Anlage 16 für ein (in Figuren 2 und 3 gezeigte) andeutungsweise gezeigtes Instrument/ Zange 12 vorgesehen, wobei die beiden (ein Paar bildende) Anlagen 16 beidseitig der Koppelstabausnehmung 14 entlang des (in Figuren 2 und 3 gezeigten) bereits eingelegten Koppelstabs 10 radial vorstehen. Da sich am ersten Rand 13 zwei diametral sich gegenüberliegende Axialschlitze 14 befinden, um den Koppelstab 10 quer zum Körper 6 und damit quer zur Montagehülse 2 aufnehmen zu können, sind bevorzugt an beiden Axialschlitzen 14 jeweils ein Anlagenpaar 16 gemäß vorstehender Ausführungen angeordnet.

Ausgehend von jeweils einem Paar Anlagen 16 bzw. vom ersten Rand 13 der Montagehülse erstreckt sich eine jeweilige Außenschräge19 axial bis zu einem zweiten Rand 18 der Montagehülse 2, der auch als vom Außengewindeschaft 4 abgewandter Rand 18 bezeichnet werden kann. Diese zwei einander bezüglich der Mittelachse 11 diametral gegenüberliegenden Außenschrägen 19 können auch als Abplattungen am Außenumfang der (zylindrischen) Montagehülse 2 bezeichnet werden. Die Außenschrägen 19 sind (trapezförmig zulaufend) derart schräggestellt, dass sie in Richtung hin zum zweiten Rand 18 aufeinander zu laufen.

Im Bereich des zweiten Rands 18 sind an jeder Außenschräge 19 zwei weitere Anlagen (Anlageflächen/Anlagekanten) 20 vorgesehen. Die beiden weiteren Anlagenpaare 20 sind nicht als Nasen gebildet, sondern fallen mit den jeweiligen Außenschrägen 19 zusammen und sind durch eine jeweilige V-Form des zweiten Randes 18 begrenzt. In anderen Worten ausgedrückt bilden die Anlagenpaare 20 an jeder Außenschräge 19 eine im Wesentlichen V-förmige Kerbe, welche ausgehend vom zweiten Rand 18 in Richtung zum ersten Rand 13 spitz zuläuft.

Figuren 2 und 3 zeigen jeweils die Pedikelschraube 1 mit der Hülse 2 und mit dem Koppelstab 10 und mit einer nur grobschematisch dargestellten Zange 12 in zwei verschiedenen Relativpositionen der Zange 12 zur Hülse 2 und damit zum Köper 6. Die Hülse 2 bietet bei beiden Relativpositionen zumindest ein Paar definierter Anlagen 16 für beide Zangentypen. Falls die nur schematisch dargestellte Zange 12 ein Kompressionsinstrument ist, ist in den Figuren 2 und 3 jeweils links eine weiter gleichartige Pedikelschraube 1 mit Hülse 2 vorgesehen, die in den Figuren 2 und 3 nach links in Richtung zur gezeigten Pedikelschraube 1 bewegt werden soll. Falls die dargestellte Zange 12 ein Distraktionsinstrument ist, ist in den Figuren 2 und 3 jeweils rechts eine weiter gleichartige Pedikelschraube 1 mit Hülse 2 vorgesehen, die in den

Figuren 2 und 3 nach rechts in Richtung weg von der gezeigten Pedikelschraube 1 bewegt werden soll.

Bei der ersten Relativposition gemäß Figur 2 überträgt die Zange 12 ihre Kraft von rechts nach links über zunächst nur das Paar nasenförmiger Anlagen 16, von denen in Figur 2 nur eine Anlage 16 zu sehen ist. Dadurch wird der nicht gezeigte Wirbel, in den die Implantatschraube 4 eingedreht ist, gemäß Fig. 2 nach links verschoben. Es erfolgt hierbei ein seitliches Ausweichen/Verschieben des Schraubenkopfs bzw. des vom Schraubenkopf gebildeten Gelenks 22, von dem in den Figuren 2 und 3 nur die Schwenk-/Schraubenkopfmittelachse gezeigt ist. Dabei verschwenkt der Körper 6 zusammen mit der Montagehülse 2 gegenüber dem Schraubenschaft 4, um sich selbstätig im Wesentlichen senkrecht zum eingelegten Koppelstab 10 auszurichten. Genauer gesagt neigt sich der von dem Außengewindeschaft 4 abgewandte (in den Figuren 2 und 3 obere) Endabschnitt der Montaghülse 2 gegen die gezeigte Branche der betroffenen Zange 12, wenn der (nicht gezeigte) Wirbel samt darin eingedrehter Implantatschraube gemäß Fig. 2 nach links gedrückt wird und dabei eine in den Körper 6 bereits lose eingedrehte Setscrew 24 längs des Koppelstabs 10 entlanggleitet.

Bei der zweiten Relativposition gemäß Figur 3 überträgt die Zange 12 ihre Kraft von rechts nach links über das Paar nasenförmiger Anlagen 16, wobei ein Verkippen des Körpers 6 in hin zur Zange 12 solange möglich ist, bis das Paar weiter Anlagen 20 am zweiten Rand 18 der Montagehülse 2 in Anlage mit der Branche der Zange 12 kommen.

Bei einem Vergleich der beiden Relativpositionen der Figuren 2 und 3 ist zu erkennen, dass bei der Krafteinleitung mittels der Zange 12 die Mittelachse 11 der Hülse 2, die im aufgesteckten Zustand der Mittelachse 11 des Körpers 6 entspricht, mit dem Körper 6 gegenüber dem Außengewindeschaft 4 verkippen kann. Dabei kann sich das Paar weiterer Anlagen 20 an der Zange 12 anlegen, so dass am Ende eine stabile Anlage zwischen der Zange 12 und der Hülse 2 gegeben ist, bei der die nötigen hohen Kräfte übertragbar sind, ohne dass die Zange 12 von der Hülse 2 abrutscht.

Die Federlaschen 9 erstrecken sich vom zweiten Rand 18 ausgehend in Richtung zum Außengewindeschaft 4 sind und dabei in jeweiligen Durchgangsausnehmungen 26 der Hülse 2 angeordnet.

Figur 4 ist der als Kugelkopf ausgebildete Gelenk-/Schraubenkopf 22, der mit dem Körper 6 ein Gelenk bildet, zusammen mit der Hülse 2 in einer längsgeschnittenen Darstellung. Es ist zu erkennen, dass der Körper 6 zwei Körperabschnitte (Tulpenflügel) 6a hat, die die Stabeinlegeöffnung 8 des Körpers 6 bilden. An Innenumfangswandungen der Körperabschnitte 6a sind Abschnitte eines Innengewindes gebildet. Das Innengewinde ist zum Einschrauben einer als Setscrew 24 bezeichneten Madenschraube geeignet und vorgesehen, um den eingelegten Koppelstab 10 in dem Körper 6 zu fixieren sowie den Körper 6 gegen den Gelenkkopf 22 zu verspannen, um deren Position relativ zueinander zu fixieren.

Der Körper 6 ist außenseitig zur lösbaren und damit übergangsweisen/temporären Befestigung der Montagehülse 2 mittels der Federlaschen 9 ausgebildet. Die Schnittebene gemäß der Fig. 4 ist durch die Federlaschen 9 gelegt, wodurch deren Formgebung zu erkennen ist. Die Federlaschen 9 haben einen nach innen gebogenen/gewölbten/vorragenden Betätigungsabschnitt 28 und einen demgegenüber radial weiter außenliegenden, freien Endabschnitt 30, an dem ein innenliegender und damit dem Körper 6 6 zugewandte Anlage-/Rastfläche 32 gebildet ist. Diese hintergreift im, auf den Körper 6 aufgesetzten Zustand der Hülse 2 eine Vertiefung 34 an der Außenwandung des Körpers 6. Diese Vertiefung 34 ist beim gezeigten Ausführungsbeispiel ein Teil einer so genannten Männchen-Einbuchtung.

Damit das Aufschieben und das anschließende Einschnappen der beiden Federlaschen 9 möglichst einfach erfolgen kann, sind Spannschrägen 36 für die Endabschnitte 30 der zugeordnete Federlaschen 9 ausgebildet, welche beim Übergleiten der Rast-/Anlageflächen (Rasthaken) 32 die Federlaschen 9 radial nach außen aufbiegen. Diese Spannschrägen 36 sind beim gezeigten Ausführungsbeispiel ebenfalls ein Teil der jeweiligen Männchen-Einbuchtungen. Die jeweilige Spannschräge 36 ist an der Außenwandung des Körpers 6 zwischen der in die Montagehülse 2 eingetauchten Stirnseite des Körpers 6 und der Vertiefung 34 des Körpers 6 gebildet.

Beim gezeigten Ausführungsbeispiel sind die beiden hülsenseitigen Anlageflächen 32 schräg zur Mittelachse 11 der Hülse 2, d.h. in Richtung zum ersten Rand 13 geneigt. Dann ist in Abhängigkeit der Schrägstellung dieser Anlageflächen 32 und der Federkraft der Federlasche 9 auch ein Lösen der Hülse 2 vom Körper 6 ohne Instrument/Werkzeug möglich.

Die Schrägstellung der beiden hülsenseitigen Anlagefläche 32 schräg zur Mittelachse 11 und die Formgebung der Außenseite des Körpers 6 und der Innenseite der Hülse 2 sind derart aufeinander abgestimmt, dass die Fixierung der Hülse 2 auf dem Körper 6 in allen sechs Raumachsen (drei translatorische und drei rotatorische) wirkt. Dazu spannen die Federlaschen 9 einen Innenrand der Hülse 2 auch axial in Richtung zum Körper 6 (in Figur 4 nach unten) gegen die Stirnseite der Körpers 6 vor.

Zwischen dem zweiten Rand 18 und dem Endabschnitt 30 der Federlasche 9 (an dem sich die Anlageflächen/Rastvorsprünge 32 ausbilden) ist der Betätigungsabschnitt 28 angeordnet oder gebildet, über den die Federlasche 9 nach außen weg von der Mittelachse 11 gedrängt werden kann, um so die Hülse 2 freizugeben und wieder vom Körper 6 zu lösen. Bei einer platzsparenden Ausgestaltung ist der Endabschnitt 30 radial weiter außen als der Betätigungsabschnitt 28 angeordnet.

Zum Lösen der Federlaschen 9 der Hülse 2 vom Körper 6 ist an der Innenseite der Hülse 2 eine Nut 38 vorgesehen, die sich in Umfangsrichtung der Hülse 2 erstreckt, und die in der (in den Figuren 2 und 3 gezeigten) Durchgangsausnehmung 26 mündet. Dabei mündet die Nut in die Durchgangsausnehmung 26 entlang der Mittelachse 11 der Hülse 2 betrachtet (in Figur 4 senkrecht) in derjenigen axialen Position oder Stelle, die der des Betätigungsabschnitts 28 entspricht.

Dann kann ein in Figur 5 gezeigter Gegenhalter 40 in Figur 5 von oben durch einen zentralen, axialen Durchgangskanal 44 der Hülse 2 in deren oberen Teil bajonettartig eingeführt und gedreht werden. Dabei werden zwei am Außenumfang des Gegenhalters 40 angeordneten Nasen 42 durch jeweils eine Nut 38 bewegt und schließlich gegen den Betätigungsabschnitt 28 jeder Federlasche 9 bewegt. Zwei weitere Nasen 42 werden gleichzeitig in die Nut 38 bewegt. Durch die beiden erstgenannten Nasen 42 werden die beiden Federlaschen 9 nach außen gedrängt, womit die Hülse 2 vom Körper 6 entriegelt wird. Durch die beiden weiteren Nasen 42 kann die Hülse 2 danach zusammen mit dem Gegenhalter 40 vom Körper 6 abgezogen werden.

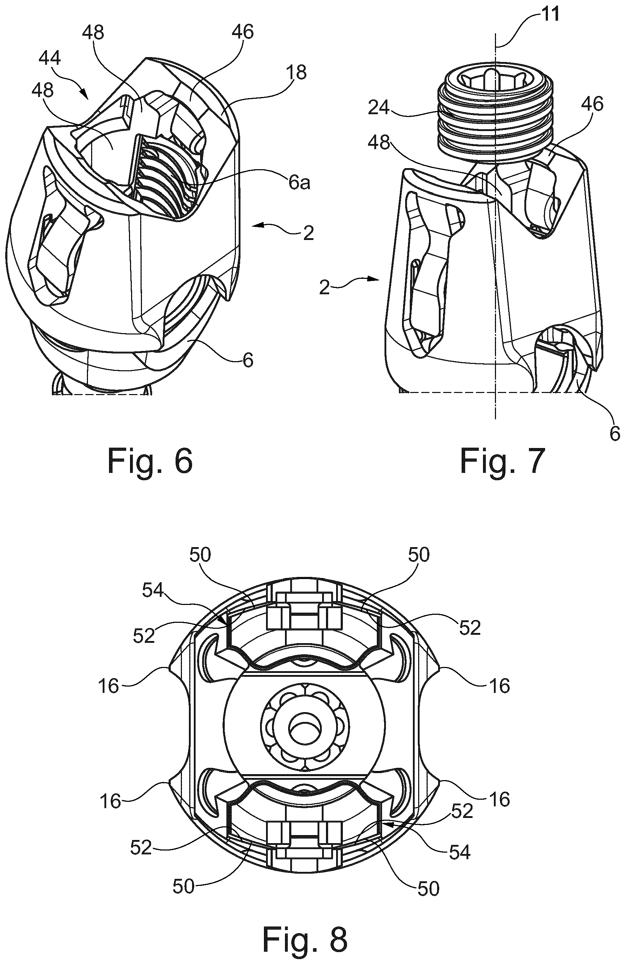

Figur 6 zeigt die Pedikelschraube 1 mit der Montagehülse 2 in einer Ansicht auf deb axialen Durchgangskanal 44 der Hülse 2. Es ist hierin einer der beiden Körperabschnitte 6a der Körpers 6 mit dem entsprechenden Innengewindeabschnitt für die (In Figur 7 gezeigte) Setscrew 24 zu erkennen. An der Hülse 2 ist am zweiten Rand 18 benachbart zu den beiden Federlaschen 9 eine jeweilige Einführschräge 46 für die Setscrew 24 vorgesehen. Diese beiden Einführschrägen 46 können auf einem gemeinsamen Konus liegen und dienen zur axialen Ausrichtung der Setscrew 24 bei deren axiale Einführung in die Montagehülse 2.

Am Innenumfang der Hülse 2 ist zwischen den beiden Körperabschnitten 6a eine jeweilige Führungsfläche 48 für die Setscrew 24 vorgesehen. Die beiden Führungsflächen 48 liegen auf einem gemeinsamen Kreiszylinder und richten die Setsrew 24 entlang der Mittalachse 11 aus, damit sie ohne zu verkanten in das vom Körper 6 gebildete Innengewinde eingeschraubt werden kann.

Figur 7 zeigt einen Teil des Körpers 6 mit der Hülse 2 mit einer Setscrew 24, bevor diese durch die Einführhilfen, genauer gesagt zunächst durch die beiden Einführschrägen 46 und dann durch die beiden Führungsflächen 48 fluchtend zur Mittelachse 11 ausgerichtet wird.

Figur 8 ist ein Querschnitt durch den Körper 6 und die Hülse 2. Da Außenwandungen 50 der Körperabschnitte 6a und innere Stützwandungen 52 der Hülse 2 teilkreiszylindrisch sind und passgenau aneinander anliegen, ist die Hülse 2 zu einer radial wirkenden Stütz- und Stabilisierungshülse (Manschette) weitergebildet.

Diese verhindert ein Auseinanderdrängen der beiden Körperabschnitte 6a, auch wenn der Koppelstab 10 (siehe Figuren 2 und 3) und die Setscrew 24 (siehe Figur 7) dazwischen gespannt bzw. gepresst werden.

Auch in rotatorischer Richtung um die Mittelachse 11 (siehe Figur 7) ist eine Verdrehsicherung 54 vorgesehen, die eine Relativdrehung der beiden Bauteile 2, 6 um die Mittelachse 11 verhindert. Weiterhin erlaubt die Verdrehsicherung 54 ein Aufsetzten der Hülse 2 auf den Körper 6 nur in zwei verschiedenen Drehpositionen, die zueinander um 180 gedreht sind.

Der erste Rand 13 kann auch als der dem Außengewindeschaft 4 zugewandte Rand der Hülse 2 bezeichnet werden. Der zweite Rand 18 kann auch als der vom Außengewindeschaft 4 abgewandte Rand der Hülse 2 bezeichnet werden. Beide Ränder 13 ,18 laufen um die Mittelachse 11 um. Die Mittelachse 11 ist bei gezeigten Ausführungsbeispiel eine Symmetrieachse.

Die Hülse 2 und/oder ein Kombiinstrument, von dem in Figur 6 nur der Gegenhalter 40 gezeigt ist, können/kann mit einem additiven Verfahren hergestellt sein.

Offenbart sind eine Hülse 2 für einen Körper 6 einer Implantatschraube 1 insbesondere für die Wirbelsäulenchirurgie und eine Implantatschraube 1 mit einem Körper 6, auf den die Hülse 2 aufgesteckt werden kann und per Schnappverbindung gesichert werden kann. Die Hülse 2 dient als definierte Anlage 16, 20 für ein Kompressionsinstrument oder für ein Distraktionsinstrument bzw. die Hülse 2 hat mindestens eine derartige Anlage 16, 20. Es können vier nasenförmige Anlagen 16 und entlang einer Mittelachse beabstandet weitere Anlagen 20 vorgesehen sein.

Die Hülse 2 hat vorzugsweise schräge und abgeplattete Bereiche 19, so dass sich Freiräume am Außenumfang der Hülse 2 ergeben, die es ermöglichen, dass die Zange 12 sich unter Schrägstellung an die Mittelachse 11 der Hülse 2 und des Körpers 6 annähern kann.

### Bezugszeichenliste:

- 1: Implantatschraube
- 2: Hülse
- 4: Außengewindeschaft
- 6: Körper
- 6a: Körperabschnitt
- 8: Stabeinlegeöffnung (des Körpers)
- 9: Federlasche
- 10: Koppelstab
- 11: Mittelachse
- 12: Zange
- 13: erster Rand
- 14: Koppelstabausnehmung (der Hülse)
- 16: Anlage
- 18: zweiter Rand
- 19: Außenschräge
- 20: weitere Anlage
- 22: Gelenk(kopf)
- 24: Setscrew
- 26: Durchgangsausnehmung (der Hülse)
- 28: Betätigungsabschnitt
- 30: Endabschnitt
- 32: Anlagefläche
- 34: Vertiefung
- 36: Spannschräge
- 38: Nut
- 40: Gegenhalter / Betätigungsstößel
- 42: Nase
- 44: Instrumentenöffnung
- 46: Einführschräge (am zweiten Rand)
- 48: Führungsfläche
- 50: Außenwandung
- 52: Stützwandung
- 54: Verdrehsicherung

## Patentansprüche

1. Montagehülse (2), die zum Aufstecken auf eine und zur lösbaren Befestigung an einer Tulpe (6) einer polyaxialen Implantatschraube (1) ausgebildet und eingerichtet ist, wofür die Montagehülse (2) in einem der Tulpe (6) zugewandten Axialabschnitt zwei diametral sich gegenüberliegende, axial sich erstreckende Wandschlitze aufweist, die gemeinsam eine quer zur Hülsenmittelachse sich erstreckende Koppelstab-Aufnahmeausnehmung (14) definieren **dadurch gekennzeichnet, dass** an einer Hülsenmantel-Außenseite der Montagehülse (2) in dem der Tulpe (6) zugewandten Axialabschnitt zumindest eine, vorzugsweise nur eine einzige sich überschneidende Axial- und Umfangsposition festgelegt ist, in der ein in Radialrichtung über den übrigen Hülsenmantel-Außenumfang nach außen vorragender Anlage- und Krafteinleitungsbereich für ein Distraktions- und/oder Kompressionsinstrument (12) ausbildet ist.

2. Montagehülse (2) nach Anspruch 1, **dadurch gekennzeichnet, dass** die Umfangsposition des Anlage- und Krafteinleitungsbereichs an der Montagehülse so gewählt ist, dass der Anlage- und Krafteinleitungsbereich in die Längsrichtung der Aufnahmeausnehmung (14), vorzugsweise ausschließlich in die Längsrichtung der Aufnahmeausnehmung (14) weist.

3. Montagehülse (2) nach Anspruch 2, **dadurch gekennzeichnet, dass** in dem zumindest einen Anlage- und Krafteinleitungsbereich ein Paar Anlagen (16) für ein Distraktionsinstrument (12) und/oder für ein Kompressionsinstrument (12) ausgebildet oder angeordnet ist, wobei die Anlagen (16) jeweils als ein von der Hülsenmantel-Außenseite radial nach außen hervorstehender Vorsprung gebildet sind, und/oder wobei die Hülsenmantel-Außenseite sich ausgehend von dem Anlage- und Krafteinleitungsbereich entlang einer Mittelachse (11) der Montagehülse (2) hin zur Mittelachse (11) verjüngt.

4. Montagehülse (2) nach einem der vorstehenden Anspruch 1 bis 3, **gekennzeichnet durch** einen ersten umlaufenden stirnseitigen Rand (13), der dafür vorgesehen ist, der Implantatschraube (1) oder ihrem Außengewindeschaft (4) zugewandt zu sein, wobei an dem ersten Rand (13) die zwei bezüglich der Mittelachse (11) einander diametral gegenüberliegenden, vorzugsweise U-förmige Wandschlitze oder Ausbuchtungen zur Durchführung oder Aufnahme eines Koppelstabes (10) in Querrichtung zur Mittelachse (11) vorgesehen sind, welche sich ausgehend vom ersten Rand (13) axial erstrecken.

5. Montagehülse (2) nach Anspruch 4, **dadurch gekennzeichnet, dass** der mindestens eine Anlage- und Krafteinleitungsbereich an dem ersten Rand (13) angeordnet oder gebildet ist.

6. Montagehülse (2) nach einem der vorhergehenden Ansprüche 1 bis 5 **dadurch gekennzeichnet, dass** zwei einander diametral gegenüberliegende Anlage- und Krafteinleitungsbereiche vorgesehen sind.

7. Montagehülse (2) nach Anspruch 3, **dadurch gekennzeichnet, dass** in dem zumindest einen Anlage- und Krafteinleitungsbereich in Umfangsrichtung gesehen beidseitig der beiden die Koppelstab-Aufnahmeausnehmung (14) definierenden Wandschlitze oder Ausbuchtungen jeweils eine Anlage (16) vorgesehen ist, wobei die beiden Anlagen (16) jedes Paares die betreffende Koppelstabausnehmung (14) oder Ausbuchtung beidseitig begrenzen.

8. Montagehülse (2) nach Anspruch 3 **dadurch gekennzeichnet, dass** die Verjüngung der Hülsenmantel-Außenseite dadurch gebildet ist, dass die Hülsenmantel-Außenseite eine kegelform oder kegelstumpfform aufweist, oder dass in den zwei Umfangsbereichen der sich diametral gegenüberliegenden Wandschlitzen trapezförmig aufeinander zulaufende Flachseiten (19) vorgesehen oder ausgebildet sind.

9. Montagehülse (2) nach Anspruch 8, **dadurch gekennzeichnet, dass** sich die Flachseiten (19) bis zu einem zweiten stirnseitigen Rand (18) erstrecken, der dem ersten Rand (13) gegenüberliegt, wobei die Flachseiten (19) in Richtung hin zum zweiten stirnseitigen Rand (18) aufeinander zulaufen.

10. Montagehülse (2) nach Anspruch 8, **dadurch gekennzeichnet, dass** an beiden Flachseiten (19) benachbart zum zweiten Rand (18) jeweils zwei weitere Anlagen (20) ausgebildet oder angeordnet sind, die vorzugsweise dadurch gebildet sind, dass der zweite Rand (18) als Begrenzung der Flachseiten oder Außenschrägen (19) jeweils eine axial sich erstreckende, vorzugsweise V-förmige Einkerbung ausbildet.

11. Montagehülse (2) nach Anspruch 8, 9 oder 10, **dadurch gekennzeichnet, dass** an dem zweiten Rand (18) trichterartige Einführschrägen (46) für eine Setscrew (24) gebildet sind, und/oder dass an einer Hülsenmantel-Innenseite teilzylindrische Führungsflächen (48) für eine Setscrew (24) gebildet sind.

12. Montagehülse (2) nach einem der vorhergehenden Ansprüche 1 bis 11, **gekennzeichnet durch** eine hülsenseitige Schnappvorrichtung, die zur lösbaren Fixierung der Montagehülse (2) auf der Tulpe (6) ausgebildet und eingerichtet ist, und die von mindestens einer Federlasche (9), vorzugsweise zwei diametral gegenüberliegenden Federlaschen (9), gebildet ist.

13. Montagehülse (2) nach Anspruch 12, **dadurch gekennzeichnet, dass** an einem freien Endabschnitt (30) der mindestens einen Federlasche (9) eine Anlagefläche (32) oder ein radial nach innen ragender Rastvorsprung zum Hintergreifen der Tulpe (6) angeordnet oder gebildet ist, wobei an der Federlasche (9) ein Betätigungsabschnitt (28) angeordnet oder gebildet ist, über den die Federlasche (9) weg von der Mittelachse (11) radial nach außen bewegbar ist.

14. Montagehülse (2) nach Anspruch 13, **dadurch gekennzeichnet, dass** die mindestens eine Federlasche (9) in einer jeweiligen schlitzförmigen Ausnehmung oder einer Durchgangsausnehmung (26) der Montagehülse (2) so angeordnet ist, dass der Betätigungsabschnitt (28) der betroffenen Federlasche (9) in deren Konstruktionslage über eine Hülsenmantel-Innenseite radial nach innen vorragt, wodurch die mindestens eine Federlasche (9) radial nach außen elastisch ausdrückbar ist.

15. Montagesystem einer oder für eine polyaxiale Implantatschraube (1), die einen gegenüber einem Außengewindeschaft (4) schwenkbare Tulpe (6) aufweist, die zwei einander gegenüberliegende Körperabschnitte (6a) oder Flanken aufweist, zwischen denen zwei U-förmige Stabeinlegeöffnungen (8) zur Aufnahme eines Koppelstabs (10) ausgebildet ist, mit einer Montagehülse (2) nach einem der Ansprüche 1 bis 14, die mittels einer Schnappvorrichtung an der Tulpe (6) derart temporär befestigbar ist, dass die beiden Körperabschnitte (6a) oder Flanken der Tulpe (6) zumindest abschnittsweise umfangsseitig stützend umfasst oder umgriffen sind, und mit einem Gegenhalter (40), der dafür ausgelegt ist, in die Montagehülse (2) eingeführt zu werden, um die wenigstens eine Federlasche (9) radial nach außen zu drücken.

16. Implantatschraubenset aufweisend:
- zumindest eine Implantatschraube (1) mit einer gegenüber einem Außengewindeschaft (4) schwenkbaren Tulpe (6), die an ihrer Außenseite wenigstens ein Rasteingriffsprofil hat oder ausbildet, sowie
- zumindest ein Montagesystem nach Anspruch 15, das darauf abgestimmt ist, dass die hülsenseitige Schnappvorrichtung von dessen Montagehülse (2) in Rasteingriff mit dem Rasteingriffsprofil des Körpers (6) zu kommen, wobei die Schnappvorrichtung der Montagehülse (2) sowie das Rasteingriffsprofil der Tulpe (6) derart aufeinander abgestimmt sind, dass bei deren in Eingriff kommen eine axiale Rasteingriffskraft zwischen der Montagehülse (2) und der Tulpe (6) erzeugt wird, welche die Montagehülse (2) axial gegen die Tulpe (6) zieht, wobei diese axiale Rasteingriffskraft vorzugsweise durch eine Schrägstellung der Anlagefläche (32) oder des Rastvorsprungs der hülsenseitigen Schnappvorrichtung mit Bezug zur Mittelachse (11) und/oder durch eine Schrägstellung des Rasteingriffsprofils der Tulpe (6) mit Bezug zu dessen Längsachse erreicht wird.
